# EUROPEAN PATENT APPLICATION

(11) **EP 2 478 959 A2**
(43) Date of publication of application: **25.07.2012**
(21) Application number: 12150896.4
(22) Date of filing: 12.01.2012
(51) Int. Cl.: B01J 23/889

(54) **Complex metal oxide catalyst, filter modiule including complex metal oxide catalyst, and air cleaner including complex metal oxide catalyst**

(30) Priority: 21.01.2011 KR 20110006489
(71) Applicant: Samsung Electronics Co., Ltd., Suwon-si, Gyeonggi-do 442-742 (KR); LeadGenex Inc., Daejeon (KR)
(72) Inventor: Kwon, Myung-jong, Gyeonggi-do (KR); Choi, Sik-sun, Gyeonggi-do (KR); Lee, Duck-hee, Seoul (KR); Bae, Jun-cheol, Gyeonggi-do (KR); Joo, Hae-ree, Gyeonggi-do (KR); Kim, Do-hoon, Daejeon (KR); Kim, Sang-woong, Daejeon (KR); Park, Sang-hyun, Daejeon (KR); Lee, Chang-ick, Chungcheongbuk-do (KR)
(74) Representative: Moy, David

(57) **Abstract**

A complex metal oxide catalyst having a composition including about 5 to about 75 wt% of manganese (Mn); about 5 to about 55 wt% of copper (Cu); and about 3 to about 60 wt% of an active compound, based on a total weight of the complex metal oxide catalyst. The complex metal oxide catalyst has excellent low-temperature activity and improved catalyst efficiency so as to be repeatedly used, and thus is effectively used in a filter module and an air cleaner.

## Description

### CROSS-REFERENCE TO RELATED APPLICATIONS

This application claims the benefit of Korean Patent Application No. 10-2011-0006489, filed on January 21, 2011, in the Korean Intellectual Property Office.

### BACKGROUND

### 1. Field

Embodiments of the present invention relate to a complex metal oxide catalyst, a filter module including the complex metal oxide catalyst, and an air cleaner including the complex metal oxide catalyst, and more particularly, to a complex metal oxide catalyst having excellent deodorizing performance and improved lifetime characteristics so as to be effectively used in various filter modules and air cleaners, a filter module including the complex metal oxide catalyst, and an air cleaner including the complex metal oxide catalyst.

### 2. Description of the Related Art

Odor causing gases consist of various compounds such as sulfurous compounds and nitrogenated compounds. Ammonia, hydrogen sulfide, and the like are main causes of odor in houses and offices. High concentration volatile organic compounds (VOCs) in combination with moisture and dust are main causes of odor in industrial settings such as a factory.

In order to remove odor or purify air in houses, an active carbon absorption process, an electric precipitation process, an ozonization process, a photocatalytic oxidation process, or the like is used. In addition, a method of removing odor or purifying air in industrial settings may be classified as a physical method such as an active carbon absorption process, a chemical method such as a chemical cleaning process, a direct combustion process, and a catalytic combustion process, or a microorganism deodorizing method such as a soil deodorization process and a carrier packed-bed microorganism deodorizing process. However, such methods are not effective in actual odorous places due to their low oxidation efficiency, high initial investment cost, and high fee pertaining to maintenance of a deodorant apparatus.

For example, the active carbon absorption process has a low initial investment cost, but has problems with costs for regenerating or changing active carbon as time passes and with secondary contamination; the direct combustion process is a relatively stable method, but needs a driving temperature of about 700 to about 900°C and thus, fuel cost are high; and the carrier packed-bed microorganism deodorizing process has a low maintenance fee, but needs a high initial investment cost and a wide installation area, and may not effectively deodorize a high-concentration odor gas.

The catalytic combustion process uses a platinum (Pt) catalyst for oxidizing main VOCs. However, since the Pt catalyst has poor low-temperature activity, is susceptible to catalyst poisoning, and requires a relatively high temperature for activating oxidization, the catalytic combustion process may not rapidly and effectively remove moisture and high-concentration inorganic odorous compounds.

Thus, there is a need for a method of rapidly adsorbing or absorbing odorous gas components, for maintaining catalyst efficiency in spite of repeated use of a catalyst as well as for easily oxidizing adsorbed gas components even at a low temperature such as room temperature.

### SUMMARY

According to the present invention there is provided a catalyst, a method of preparing a catalyst, a filter module, an air cleaner, and a method of removing a harmful substance, as set forth in the appended claims. Other features of the invention will be apparent from the dependent claims and the description which follows.

Additional aspects and/or advantages will be set forth in part in the description which follows and, in part, will be apparent from the description, or may be learned by practice of the invention.

Embodiments of the present invention provide a complex metal oxide catalyst having excellent low-temperature activity, excellent efficiencies of dissolving an odor gas and volatile organic compounds (VOCs), and improved lifetime characteristics.

Embodiments of the present invention also provide a filter module including the complex metal oxide catalyst.

Embodiments of the present invention also provide an air cleaner including the complex metal oxide catalyst.

According to an aspect of the present invention, there is provided a complex metal oxide catalyst having a composition including about 5 to about 75 wt% of manganese (Mn); about 5 to about 55 wt% of copper (Cu); and about 3 to about 60 wt% of an active compound, based on a total weight of the complex metal oxide catalyst, wherein the active compound includes at least one selected from the group consisting of cerium (Ce), ruthenium (Ru), aluminium (AI), silicon (Si), magnesium (Mg), sulfur (S), sodium (Na), and tungsten (W).

An amount of oxygen of the complex metal oxide catalyst may be about 15 to about 70 wt%, based on the total weight of the complex metal oxide catalyst.

The complex metal oxide catalyst may include about 2 to about 50 wt% of a carrier, based on the total weight of the complex metal oxide catalyst.

The complex metal oxide catalyst may have an average diameter of about 1 to about 100 nm.

A Brunner Emmett Teller (BET) surface area of the complex metal oxide catalyst may be about 200 to about 1000 m²/g.

According to another aspect of the present invention, there is provided a method of preparing a complex metal oxide catalyst, the method including dissolving and heating a manganese (Mn) precursor, a copper (Cu) precursor, and a precursor of an active compound in water in presence of a base to obtain a mixture, wherein the active compound includes at least one selected from the group consisting of cerium (Ce), ruthenium (Ru), aluminium (Al), silicon (Si), magnesium (Mg), sulfur (S), sodium (Na), and tungsten (W); putting the obtained mixture in a reactor, and aging the obtained mixture while pressing the obtained mixture at a predetermined pressure; filtering and drying the aged material; and sintering the dried material.

According to another aspect of the present invention, there is provided a filter module including the complex metal oxide catalyst; a filter support for supporting the complex metal oxide catalyst; and a heating device for supplying heat to the filter support.

According to another aspect of the present invention, there is provided an air cleaner including the filter module.

### BRIEF DESCRIPTION OF THE DRAWINGS

The above and other features and advantages of the present invention will become more apparent by describing in detail exemplary embodiments thereof with reference to the attached drawings in which:
FIG 1 is a perspective view of a filter module including a complex metal oxide catalyst, according to an embodiment of the present invention;
FIG. 2 is a diagram for describing a heating system of a filter support to which a catalyst in a powder state is fixed, according to an embodiment of the present invention;
FIG. 3 is a graph showing removing performances of a complex metal oxide catalyst of Example 1 and an active carbon catalyst of Comparative Example 1 with respect to acetaldehyde;
FIG 4 is a graph showing adsorbing performances of an active carbon catalyst of Comparative Example 1 and complex metal oxide catalysts of Examples 1 through 4 with respect to acetaldehyde;
FIG 5 is a graph showing dissolving performances of an active carbon catalyst of Comparative Example 1 and complex metal oxide catalysts of Examples 1 through 4 with respect to acetaldehyde; and
FIG. 6 is a graph showing performances with respect to repeated use of an active carbon catalyst of Comparative Example 1 and complex metal oxide catalysts of Examples 1 and 2 with respect to a complex gas.

### DETAILED DESCRIPTION

Hereinafter, embodiments of the present invention will be described in detail by explaining exemplary embodiments thereof with reference to the attached drawings.

According to an embodiment of the present invention, a manganese (Mn)-copper (Cu) based oxide catalyst, compared to a noble metal catalyst, has a low cost in regard to raw materials and has a low initialization temperature for a catalyst oxidization reaction. In addition, the Mn-Cu based oxide catalyst is easily doped with catalyst additives, and has a crystallize structure that varies according to process conditions. Thus, the Mn-Cu based oxide catalyst may be easily formed as a nano particle with a wide surface area.

A complex metal oxide catalyst formed by adding an active compound to the Mn-Cu based oxide catalyst has a composition of about 5 to about 75 wt% of Mn, about 5 to about 55 wt% of Cu, and about 3 to about 60 wt% of the active compound, based on a total weight of the complex metal oxide catalyst. The active compound may be at least one selected from the group consisting of cerium (Ce), ruthenium (Ru), aluminum (AI), silicon (Si), magnesium (Mg), sulfur (S), sodium (Na), and tungsten (W).

The complex metal oxide catalyst having the above-described composition may have improved low-temperature activity, increased activity surface area, and increased active sites and thus may have increased efficiencies of removing odor and volatile organic compounds (VOCs).

The complex metal oxide catalyst includes Mn, Cu, and the active compound. The amount of Mn may be about 5 to about 75 wt%, for example, about 25 to about 45 wt%, based on the total weight of the complex metal oxide catalyst.

The amount of Cu included in the complex metal oxide catalyst may be about 5 to about 55 wt%, for example, about 5 to about 25 wt%, based on the total weight of the complex metal oxide catalyst.

The active compound included in the complex metal oxide catalyst is used to Improve selectivity for a target material to be removed, to prevent the complex metal oxide catalyst from being poisoned, and to maintain crystalline stability, and may be, for example, at least one selected from the group consisting of Ce, Ru, Al, Si, Mg, S, Na, and W, but is not limited thereto. The amount of the active compound may be about 3 to about 60 wt%, for example, about 5 to about 25 wt%, based on the total weight of the complex metal oxide catalyst.

The complex metal oxide catalyst includes oxygen. The amount of oxygen may be about 15 to about 75 wt%, for example, about 25 to about 45 wt%, based on the total weight of the complex metal oxide catalyst.

The complex metal oxide catalyst may further include a carrier in order to improve performance of selectively removing or adsorbing a predetermined material. Examples of the carrier may include alumina, zeolite, silica, and the like. The amount of the carrier may be about 2 to about 50 wt% based on the total weight of the complex metal oxide catalyst. The complex metal oxide catalyst may be smoothly coupled to the carrier through pre-heat treatment for about 1 to about 12 hours at a temperature of about 200 to about 800°C.

The complex metal oxide catalyst may have an average diameter of about 1 to about 100 nm, for example, about 3 to about 50 nm, in order to provide sufficient catalyst activity.

In addition, the complex metal oxide catalyst may have a high specific surface area so as to provide sufficient catalyst activity. For example, a Brunner Emmett Teller (BET) surface area of the complex metal oxide catalyst may be in a range of about 200 to about 1,000 m²/g.

The complex metal oxide catalyst may remove acetaldehyde, ammonia, acetic acid, triethylamine, and the like at a temperature equal to or less than about 104°C, for example, at a temperature from room temperature to about 100°C.

The complex metal oxide catalyst may be prepared by using various methods, for example, may be prepared by using the following method.

First, the complex metal oxide catalyst may be prepared by dissolving and heating a precursor of Mn, a precursor of Cu, and a precursor of an active compound in water in presence of a base to form a mixture solution in which a precipitate in a gel state is formed, putting the mixture in a reactor and aging the mixture for a predetermined period of time while pressing the mixture at a predetermined pressure, and sintering a powder obtained by removing moisture from the aged material.

In the method of preparing the complex metal oxide catalyst, examples of the precursors of Mn, Cu, and the active compound may include carbonates, sulphates, nitrates, chlorides, nitrides, and sulfides of these foregoing metals.

Examples of the base used in the method of preparing the complex metal oxide catalyst may include sodium carbonate, sodium hydroxide, calcium carbonate, potassium carbonate, calcium hydroxide, potassium hydroxide, and the like.

Examples of the active compound used in the method of preparing the complex metal oxide catalyst may include at least one selected from the group consisting of Ce, Ru, AI, Si, Mg, S, Na, and W.

The complex metal oxide catalyst may include a carrier such as alumina, silica, and/or zeolite, thereby being a supported-type complex metal oxide catalyst The supporting method may be an impregnating process or a power mixing process, but is not limited thereto.

The impregnating method may include preparing a carrier having a circle or pellet shape, aging and sintering the carrier together with the mixture solution in which the precipitate in a gel state is formed, and then supporting the carrier.

The power mixing process may include sintering and supporting a mixture obtained by mixing a resulting powder obtained through aging and drying processes performed during manufacture of the complex metal oxide catalyst with a carrier In a powder state.

In the method of preparing the complex metal oxide catalyst, the sintering may be performed while a pressure of the reactor is maintained at about 1 to about 100 atm, in order to increase a surface area and to form nano pores. The sintering may be performed for about 1 to about 10 hours.

Since the active compound is used, and the sintering is performed at a pressure equal to or greater than atmospheric pressure, the number of catalyst active sites that protrude from a surface of the complex metal oxide catalyst through nano-sized or micro-sized pores is increased. Thus, the surface area of the complex metal oxide catalyst and the number of active sites are increased, and thus the complex metal oxide catalyst may have improved efficiencies of removing odor and VOCs and improved low-temperature catalyst activity.

After the aging is performed, water or an organic solvent is used to wash the resulting material and then impurities are filtered from the resulting material in order to remove the impurities, and then the resulting material is dried to remove moisture. Then, the sintering is performed for about 1 to about 10 hours at a temperature of about 250 to about 600°C.

The complex metal oxide catalyst prepared by using the above-described process may have an average diameter of about 1 to about 100 nm. The BET surface area of the complex metal oxide catalyst may be in the range of about 200 to about 1,000 m²/g.

The complex metal oxide catalyst may have a pellet or granule shape. To this end, a forming process may be performed. The forming process may be performed prior to the sintering. In order to perform the forming process, various binders may be used.

According to another embodiment of the present invention, the complex metal oxide catalyst may be used in a filter module, and the filter module is installed in an air cleaner. The filter module may be configured as follows.

The filter module may include the complex metal oxide catalyst, a filter support for supporting the complex metal oxide catalyst, and a heating device for supplying heat to the filter support.

The complex metal oxide catalyst has been described above.

The filter support may include a metal, ceramic, glass that has heat resistance, and may have a form, honeycomb, fiber, or corrugated form. For example, the filter support may have excellent thermal conductivity by using, for example, a Cu, Ni, and/or Fe metal form, Al in the form of a honeycomb, and SIC in the form of a honeycomb form. In addition, the filter support may include the complex metal oxide catalyst in order to maximize catalyst activity and heat transfer.

The complex metal oxide catalyst in a powder state may be fixed to the filter support by using a silica binder at a temperature of about 100°C or more, for example, a temperature of about 100 to about 300°C, or may have a pellet shape and be filled in the filter support.

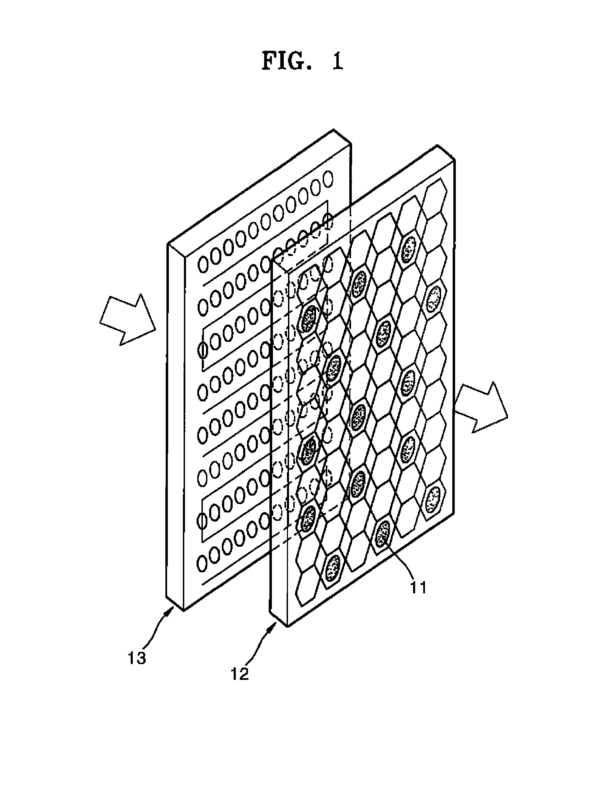

According to an embodiment of the present invention, as shown in FIG 1, a complex metal oxide catalyst 11 having a pellet shape is filled in an Al honeycomb filter support 12. In addition, a heating system 13 is configured to be able to be attached to or separated from the Al honeycomb filter support 12 so as to directly or indirectly heat the Al honeycomb filter support 12 to transfer heat to the complex metal oxide catalyst 11. In this case, in order to maximize transfer of heat to the complex metal oxide catalyst 11, the complex metal oxide catalyst 11 may be put in the heating system 13 and thus the complex metal oxide catalyst 11 and the heating system 13 may be integrated with each other.

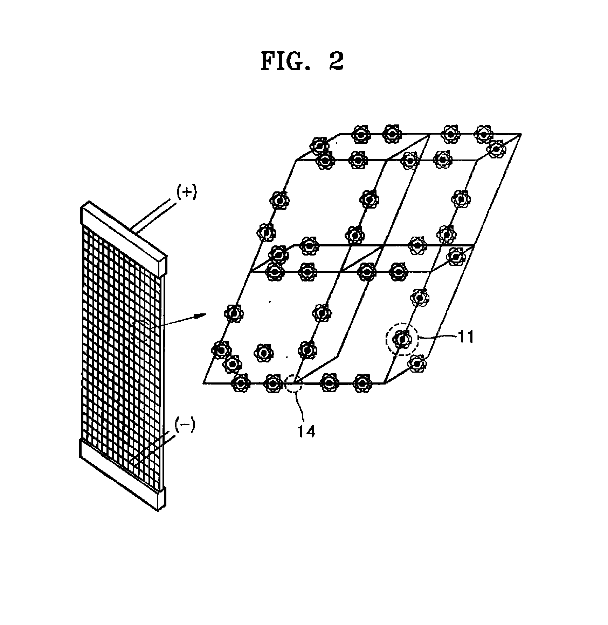

The heating system 13 may directly heat a filter frame 14 of the Al honeycomb filter support 12 to which the complex metal oxide catalyst 11 in a powder state is fixed by resistance heating, as shown in FIG 2.

The above-described filter module may be effectively used in various types of home or industrial air cleaners.

Hereinafter, one or more embodiments of the present invention will be described in detail with reference to the following examples. However, these examples are not intended to limit the purpose and scope of the one or more embodiments of the present invention.

### Examples 1

A first solution was prepared by dissolving 462 g of MnSO₄·5H₂O and 318 g of Na₂CO₃ in 2 L of distilled water. A second solution was prepared by dissolving 75 g of CeCl₃-7H₂O and 136 g of CuCl₂-2H₂O In 1 L of distilled water. Then, the first solution and the second solution were each stirred for 30 minutes at a temperature of 90°C. Then, the first solution and the second solution were put in a steel reactor, and were aged for 4 hours at a constant pressure of 5 atm. The pressure of the steel reactor was lowered to atmospheric pressure to obtain a mixture solution in a gel state, and a temperature of the steel reactor was cooled to room temperature to obtain a cooled material in a gel state.

The cooled material in a gel state was washed and filtered with deionized water, and was heated in an oven for 12 hours at a temperature of 60°C to remove moisture. A complex metal oxide catalyst was prepared by sintering a forming material obtained by forming the dried material to have a pellet shape for 3 hours at a temperature of 300°C.

### Example 2

A first solution was prepared by dissolving 462 g of MnSO₄·5H₂O and 318 g of Na₂CO₃ in 2 L of distilled water. A second solution was prepared by dissolving 75 g of CeCl₃·7H₂O and 136 g of CuCl₂·2H₂O in 1 L of distilled water. Then, the first solution and the second solution were each stirred for 30 minutes at a temperature of 90°C. Then, the first solution and the second solution were put in a steel reactor, and were aged for 4 hours at a constant pressure of 5 atm. The pressure of the steel reactor was lowered to atmospheric pressure to obtain a mixture solution in a gel state, and a temperature of the steel reactor was cooled to room temperature to obtain a cooled material in a gel state.

The cooled material in a gel state was washed and filtered with deionized water, and was heated in an oven for 12 hours at a temperature of 60°C to remove moisture, thereby obtaining a complex metal oxide catalyst having a particle shape. The complex metal oxide catalyst and particles of an alumina carrier were uniformly mixed, and were formed together to have a pellet shape, thereby preparing a forming material. A complex metal oxide catalyst including 20 wt% of the alumina carrier based on a total weight of the complex metal oxide catalyst was prepared by sintering the forming material for 3 hours at a temperature of 300°C.

### Example 3

A complex metal oxide catalyst was prepared in the same manner as In Example 2 except that the complex metal oxide catalyst included 40 wt% of the alumina carrier based on the total weight of the complex metal oxide catalyst.

### Example 4

A complex metal oxide catalyst including 20 wt% of a zeolite carrier was prepared in the same manner as in Example 2 except that the zeolite carrier was used instead of the alumina carrier.

### Example 5

A first solution was prepared by dissolving 462 g of MnSO₄·5H₂O and 318 g of Na₂CO₃ in 2 L of distilled water. A second solution was prepared by dissolving 75 g of CeCl₃·7H₂O and 136 g of CuCl₂·2H₂O in 1 L of distilled water. Then, the first solution and the second solution were each stirred for 30 minutes at a temperature of 90°C. Then, the first solution and the second solution were put in a steel reactor, and were aged for 4 hours at a constant pressure of 5 atm. The pressure of the steel reactor was lowered to atmospheric pressure to obtain a mixture solution in a gel state, and a temperature of the steel reactor was cooled to room temperature to obtain a cooled material in a gel state.

The cooled material in a gel state was washed and filtered with deionized water, and was heated in an oven for 12 hours at a temperature of 60°C to remove moisture. A complex metal oxide catalyst having a particle shape was prepared by sintering the dried material for 3 hours at a temperature of 300°C.

The complex metal oxide catalyst had an average diameter of 5 nm and had a BET surface area of 220 m²/g.

### Example 6: Preparation of filter module

Like in FIG. 1, a filter module was prepared by filing the complex metal oxide catalyst having a pellet shape prepared in Example 1, 2, 3 or 4, in an Al honeycomb filter support, preparing a separate heater for supplying heat to the Al honeycomb filter support, and attaching the separate heater to the Al honeycomb filter support.

### Comparative Example 1

Like in FIG 1, a filter module was prepared by filling an active carbon catalyst having a pellet shape in a honeycomb filter support, preparing a separate heater for supplying heat to the Al honeycomb filter support, and attaching the separate heater to the Al honeycomb filter support.

### Evaluation Example

Removing performance and deodorizing performance with respect to repeated use of the filter module of Example 6 including the complex metal oxide catalyst of Example 1 were tested with respect to temperature and time by using a chamber method using acetaldehyde, acetic acid, and ammonia independently or in combinations of two or more.

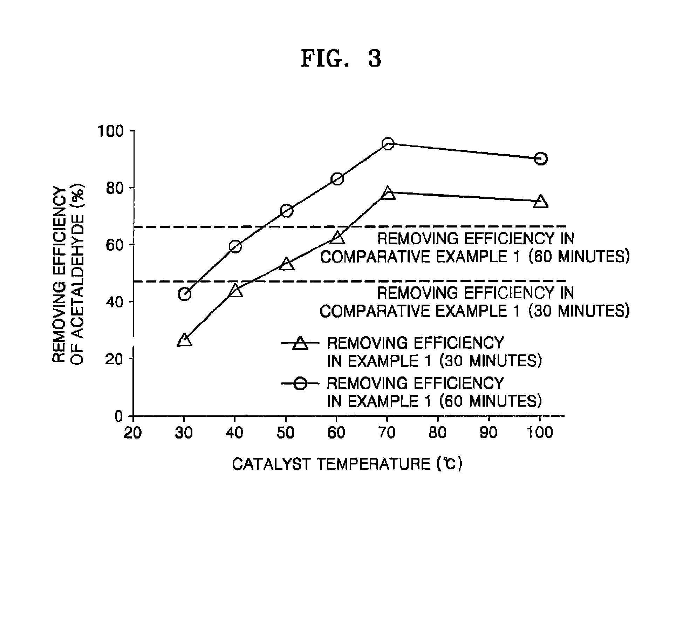

FIG. 3 is a graph showing removing performances of the complex metal oxide catalyst of Example 1 and the active carbon catalyst of Comparative Example 1 with respect to acetaldehyde. The complex metal oxide catalyst had higher removing performance at a temperature of 50°C than the active carbon catalyst, had saturated catalyst activity at a temperature of 70°C, and removed acetaldehyde by 90 % or more within 60 minutes.

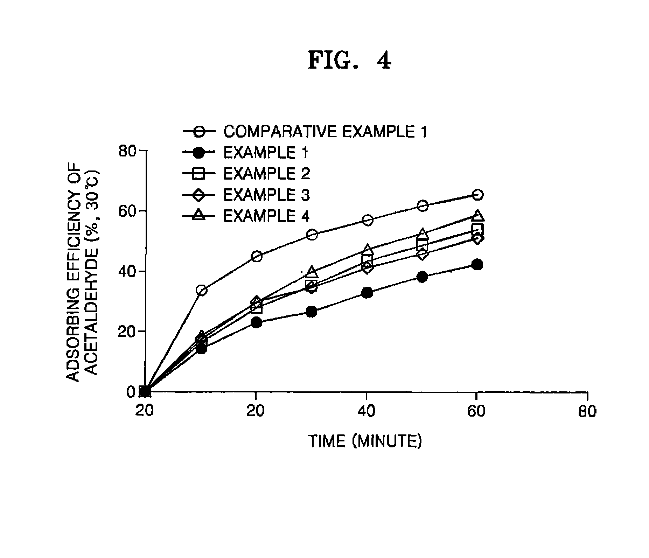

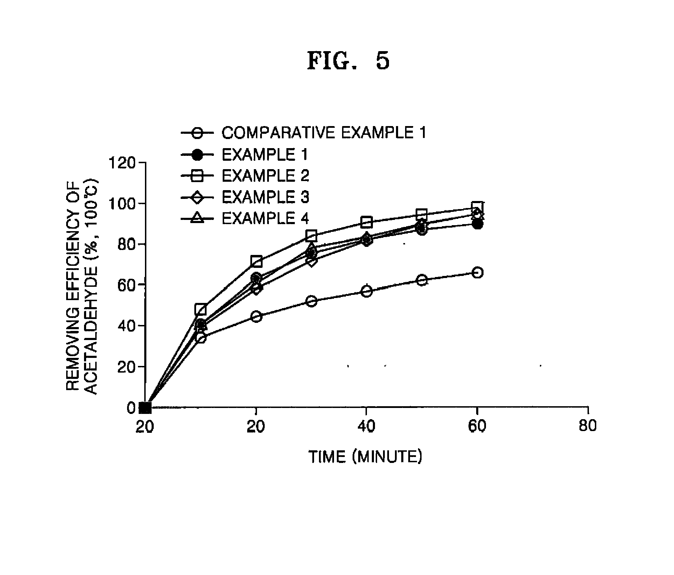

FIGS. 4 and 5 are graphs showing adsorbing and dissolving performances of the active carbon catalyst of Comparative Example 1 and the complex metal oxide catalysts of Examples 1 through 4 with respect to acetaldehyde. When a complex metal oxide catalyst includes a carrier had increased adsorbing performances, and the dissolving performances thereof were maintained to have a current level or more. When a predetermined amount of a carrier is added to the complex metal oxide catalyst according to an embodiment of the present invention, adsorbing performance of the complex metal oxide catalyst is improved while dissolving performance of the complex metal oxide catalyst is maintained. Thus, the complex metal oxide catalyst may be used to adsorb a predetermined material.

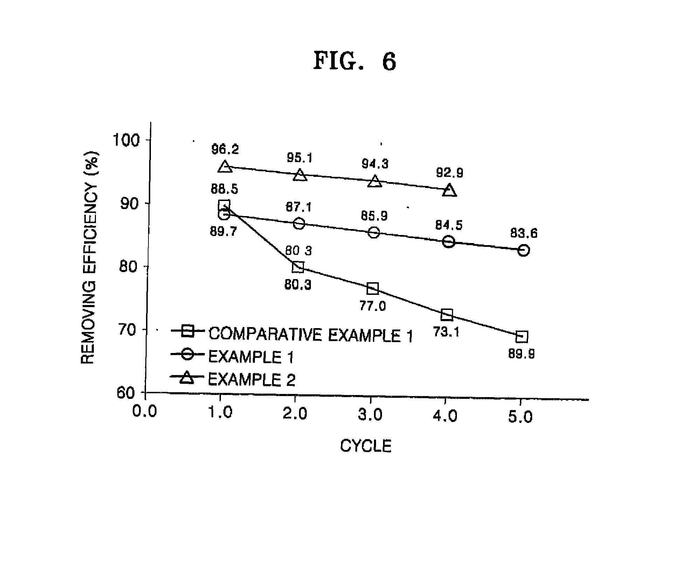

FIG. 6 is a graph showing results of an experiments that was repeated 5 times using filter modules including the active carbon catalyst of Comparative Example 1, the complex metal oxide catalyst (90%) of Example 1, and the complex metal oxide catalyst/alumina (weight ratio of 8/2, 90%) of Example 2 and a complex gas of acetic acid and acetaldehyde. Removing efficiency of the active carbon catalyst gradually reduced as the experiment was repeated. After the experiment was repeated 5 times, the removing efficiency of the active carbon catalyst reduced by 22%. Removing efficiencies of the complex metal oxide catalyst of Example 1 and the complex metal oxide catalyst including alumina of Example 2 reduced by 6% or less after the experiment was repeated 5 times. Thus, the complex metal oxide catalyst of Example 1 and the complex metal oxide catalyst including alumina of Example 2 had performances with respect to repeated use 4 times or more higher than that of the active carbon catalyst.

In addition, the complex metal oxide catalyst including alumina of Example 2 for selectively removing ammonia had removing performance of 90 % or more with respect to the complex gas, and had 9% higher performance than the complex metal oxide catalyst By adding a carrier to the complex metal oxide catalyst according to an embodiment of the present invention, a predetermined material may be selectively removed.

The complex metal oxide catalyst according to an embodiment of the present invention may have excellent low-temperature activity, excellent efficiencies of dissolving an odor gas and VOCs, and Improved lifetime characteristics, and thus may be effectively used in a filter module or an air cleaner.

While the present invention has been particularly shown and described with reference to exemplary embodiments thereof, it will be understood by those of ordinary skill in the art that various changes in form and details may be made therein without departing from the spirit and scope of the present invention as defined by the following claims.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed Is one example only of a generic series of equivalent or similar features.

The invention is not restricted to the details of the foregoing embodfment(s). The invention extends to any novel one, or any novel combination, of the features disclosed in this specification (including any accompanying claims, abstract and drawings), or to any novel one, or any novel combination, of the steps of any method or process so disclosed.

## Claims

1. A complex metal oxide catalyst having a composition comprising:
about 5 to about 75 wt% of manganese (Mn); about 5 to about 55 wt% of copper (Cu);
and about 3 to about 60 wt% of an active compound, based on a total weight of the complex metal oxide catalyst,
wherein the active compound comprises at least one selected from the group consisting of cerium (Ce), ruthenium (Ru), aluminium (Al), silicon (Si), magnesium (Mg), sulfur (S), sodium (Na), and tungsten (W).

2. The complex metal oxide catalyst according to claim 1, wherein the complex metal oxide catalyst comprises about 2 to about 50 wt% of a carrier, based on the total weight of the complex metal oxide catalyst.

3. The complex metal oxide catalyst according to preceding claims, wherein an amount of oxygen of the complex metal oxide catalyst is about 15 to about 70 wt%, based on the total weight of the complex metal oxide catalyst.

4. The complex metal oxide catalyst according to preceding claims, wherein the carrier comprises at least one selected from the group consisting of silica, zeolite, and alumina.

5. The complex metal oxide catalyst according to preceding claims, wherein a Brunner Emmett Teller (BET) surface area of the complex metal oxide catalyst is about 200 to about 1000 m²/g.

6. The complex metal oxide catalyst according to preceding claims, wherein the amount of Mn is about 25 to about 45 wt%, the amount of Cu is about 5 to about 55 wt%, the amount of the active compound is about 5 to about 25 wt%, and an amount of oxygen is about 25 to about 45 wt%.

7. A method of preparing a complex metal oxide catalyst, the method comprising:
dissolving and heating a manganese (Mn) precursor, a copper (Cu) precursor, and a precursor of an active compound in water in presence of a base to obtain a mixture, wherein the active compound comprises at least one selected from the group consisting of cerium (Ce), ruthenium (Ru), aluminium (Al), silicon (Si), magnesium (Mg), sulfur (S), sodium (Na), and tungsten (W);
putting the obtained mixture in a reactor, and aging the obtained mixture while pressing the obtained mixture;
filtering and drying the aged material; and
sintering the dried material.

8. The method according to claim 7, wherein the aging comprises putting a carrier together with the obtained mixture in the reactor.

9. The method according to preceding claims, further comprising:
after the drying, preparing a mixed powder by mixing the dried material and a carrier in a powder state.

10. A filter module comprising:
a complex metal oxide catalyst comprising;
about 5 to about 75 wt% of manganese (Mn); about 5 to about 55 wt% of copper (Cu); and about 3 to about 60 wt% of an active compound, based on a total weight of the complex metal oxide catalyst,
wherein the active compound comprises at least one selected from the group consisting of cerium (Ce), ruthenium (Ru), aluminium (Al), silicon (Si), magnesium (Mg), sulfur (S), sodium (Na), and tungsten (W);a filter support for supporting the complex metal oxide catalyst; and
a heating device for supplying heat to the filter support.

11. The filter module according to claim 10, wherein the complex metal oxide catalyst in a powder state is fixed to the filter support by using a silica binder at a temperature of about 100°C or more.

12. The filter module according to preceding claims, wherein the filter support comprises the complex metal oxide catalyst.

13. The filter module according to preceding claims, wherein the heating device directly heats the filter support so as to transfer heat to the complex metal oxide catalyst.

14. An air cleaner comprising:
a filter module;
a complex metal oxide catalyst comprising;
about 5 to about 75 wt% of manganese (Mn); about 5 to about 55 wt% of copper (Cu); and about 3 to about 60 wt% of an active compound, based on a total weight of the complex metal oxide catalyst,
wherein the active compound comprises at least one selected from the group consisting of cerium (Ce), ruthenium (Ru), aluminium (Al), silicon (Si), magnesium (Mg), sulfur (S), sodium (Na), and tungsten (W);
a filter support for supporting the complex metal oxide catalyst; and
a heating device for supplying heat to the filter support.

15. A method of removing a harmful substance, the method comprising:
removing an odor gas or volatile organic compounds (VOCs) at a temperature of about 100°C or less by using a filter module wherein the filter module comprises:
a complex metal oxide catalyst comprising;
about 5 to about 75 wt% of manganese (Mn); about 5 to about 55 wt% of copper (Cu); and about 3 to about 60 wt% of an active compound, based on a total weight of the complex metal oxide catalyst,
wherein the active compound comprises at least one selected from the group consisting of cerium (Ce), ruthenium (Ru), aluminium (Al), silicon (Si), magnesium (Mg), sulfur (S), sodium (Na), and tungsten (W);
a filter support for supporting the complex metal oxide catalyst; and
a heating device for supplying heat to the filter support.
